# EUROPEAN PATENT APPLICATION

(11) **EP 4 574 135 A1**
(43) Date of publication of application: **25.06.2025**
(21) Application number: 24222149.7
(22) Date of filing: 20.12.2024
(51) Int. Cl.: A61K 9/00, A61K 9/20, A61K 31/519

(54) **TABLETS COMPRISING BREXPIPRAZOLE**

(30) Priority: 22.12.2023 TR 202318024
(71) Applicant: Sanovel Ilac Sanayi Ve Ticaret Anonim Sirketi, Istanbul (TR)
(72) Inventor: SUNEL, Fatih, Istanbul (TR); ATAK, Fadime Bilgehan, Istanbul (TR); ATAMAN, Seval, Istanbul (TR); SARP, Onder, Istanbul (TR)
(74) Representative: Genç Ilhan, Oznur

(57) **Abstract**

The present invention relates to a tablet comprises brexpiprazole or a salt thereof and at least two fillers wherein the one of fillers is corn starch and brexpiprazole has a d (0.9) particle size between 1 µm and 35 µm. Further, the present invention also relates to a simple, rapid, cost effective, timesaving and industrially convenient process.

## Description

### Field of the Invention

The present invention relates to a tablet comprises brexpiprazole or a salt thereof and at least two fillers wherein the one of fillers is corn starch and brexpiprazole has a d (0.9) particle size between 1 µm and 35 µm. Further, the present invention also relates to a simple, rapid, cost effective, timesaving and industrially convenient process.

### Background of the Invention

Brexpiprazole acts as a partial agonist of the serotonin 5-HT1A receptor and the dopamine D2 and D3 receptors. Brexpiprazole has been described for use in the treatment of schizophrenia, depression and other central nervous system disorders, and for use in the prophylaxis and/or treatment of behavioral and psychological symptoms associated with neurodegenerative disease or impulsive symptoms associated with mental disease.

Brexpiprazole is a compound represented by the following Formula I, and its chemical name is 7-[4-[4-(1-benzothiophen-4-yl)piperazin-1-yl]butoxy]-1H-quinolin-2-one.

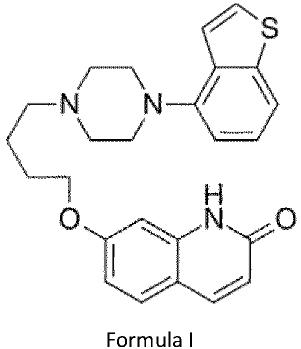

Brexpiprazole is an antidepressant and antipsychotic drug marketed under the brand Rexulti^{®} for the oral treatment of schizophrenia and as an adjunctive treatment to antidepressants in major depressive disorder. REXULTI tablets are intended for oral administration and available in 0.25 mg, 0.5 mg, 1 mg, 2 mg, 3 mg and 4 mg strengths. The product was approved in the U.S. in 2015 for the aforementioned indications and is currently in phase III trials for the treatment of agitation associated with Alzheimer's disease and the treatment of PTSD (post-traumatic stress disorder).

Brexpiprazole has poor solubility in water. The major limitation of the drug is its low solubility. So, in a formulation comprising brexpiprazole, solubility is important.

WO2019121849 patent application relates to a pharmaceutical composition in the form of a tablet, comprising a) brexpiprazole as active ingredient and b) at least 5% by weight (wt%) pregelatinized starch (PGS). The invention relates to methods of preparing the tablet comprising dry granulation of a blend of the components to produce granules and compression of the granules to a tablet.

EP3545950A1 patent application relates to a pharmaceutical composition comprising a granulate comprising brexpiprazole, wherein the granulate is obtained by wet-granulation of a carrier using a granulation liquid that is a solution of brexpiprazole in a solvent system.

In prior art, low solubility of brexpiprazole has been approached in several ways. However, we have seen that studies carried out to correct the dissolution profile may cause some stability problems.

There is thus still a need for a film coated tablet comprising brexpiprazole or a salt thereof that provides a tablet having the desired stability, dissolution profile, hardness and compressibility, in another words the disadvantages seen in the active substance will able to overcome. The formulation has been developed by using standard techniques which is simple and cost-effective method.

### Detailed Description of the Invention

The main object of the present invention is to eliminate problems caused by active substance, brexpiprazole, and bringing additional advantages to the relevant prior art.

Another object of the present invention is to provide a tablet comprising brexpiprazole with content uniformity and compressibility and having desired level of dissolution profile.

Another object of the present invention is to provide a tablet comprising brexpiprazole having improved high stability.

We have found that brexpiprazole or a salt thereof having the following particle sizes is very important for formulation. Especially, it positively affects the dissolution properties and powder homogenization. The obtained tablets have the desired dissolution profile. The powder is more homogeneous. The content uniformity of the tablets obtained from the more homogeneous powder is more ideal. Therefore, it provides better bioavailability. In addition to this having corn starch as filler helps to provide the desired dissolution profile, also corn starch ensures that the tablet does not break easily in the formulation.

As used here in, 'particle size' means the cumulative volume size distrubition as tested by any conventionally accepted method such as the laser diffraction method (i.e. Malvern Mastersizer 2000 analysis). The term d (0.9) means the size at which %90 by volume of the particles are finer.

According to one embodiment of the present invention, a tablet comprises brexpiprazole or a salt thereof and at least two fillers wherein the one of fillers is corn starch and brexpiprazole has a d (0.9) particle size between 1 µm and 35 µm.

In one embodiment of the invention, brexpiprazole or a salt thereof has a d (0.9) particle size between 3 µm and 28 µm, more preferably between 5 µm and 18 µm.

According to one embodiment of the present invention, the amount of brexpiprazole or a salt thereof is between 0.1% and 10.0% by weight of the total composition.

According to one embodiment of the present invention, used another fillers are selected from group comprising lactose monohydrate, microcrystalline cellulose, mannitol, pregelatinized starch, ammonium alginate, calcium carbonate, anhydrous lactose, calcium phosphate, calcium phosphate dehydrate, neutral pellets, calcium sulfate, cellulose, ethylcellulose, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals, talc, polysorbate 80, xylitol or mixtures thereof.

According to this embodiment of the present invention, another filler is lactose monohydrate, microcrystalline cellulose or mixtures thereof.

According to this embodiment of the present invention, another filler is lactose monohydrate and microcrystalline cellulose.

According to this embodiment of the present invention, the amount of filler in the tablet is 80.0% to 95.0% by weight of the total composition. Preferably, it is 85.0% to 92.0% by weight of the total composition.

According to this embodiment of the present invention, the tablet further comprises at least one pharmaceutically acceptable excipient which is selected from the group comprising binders, disintegrants, lubricants or mixtures thereof.

Suitable binders are selected from the group comprising polyvinylpyrrolidone, carboxymethylcellulose sodium, sugars, agar, alginates, carbomers, cellulose acetate phthalate, chitosan, starch, starch mucilage, acacia mucilage, dextrates, dextrin, dextrose, ethylcellulose, glyceryl behenate, hydrogenated vegetable oil type I, hydroxyethyl cellulose, hydroxyethylmethyl cellulose, hydroxypropyl cellulose, hydroxypropyl starch, hypromellose, magnesium aluminum silicate, maltodextrin, methylcellulose, poloxamer, polycarbophil, polydextrose, polyethylene oxide, polymethacrylates, aluminia hydroxide, stearic acid, sucrose, , polyoxyethilene-alkyl ethers, pullulan or mixtures thereof.

According to this embodiment of the present invention, the binder is polyvinylpyrrolidone or hydroxypropyl cellulose or mixtures thereof.

According to this embodiment of the present invention, the amount of binder in the tablet is 1.0% to 10.0% by weight of the total composition. Preferably, it is 2.0% to 6.0% by weight of the total composition.

Suitable disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, alginic acid, magnesium aluminium silica, poloxamer, sodium glycine carbonate or mixtures thereof.

According to this embodiment of the present invention, the disintegrant is crospovidone. This helps to provide the desired dissolution profile.

According to this embodiment of the present invention, the amount of disintegrant in the tablet is 1.0% to 10.0% by weight, preferably 2.0% to 4.6% by weight of the total composition.

Suitable lubricants are selected from the group comprising sodium stearyl fumarate, magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate or mixtures thereof.

According to this embodiment of the present invention, the lubricant is sodium stearyl fumarate. It improves the powder processing properties of tablet formulation. Also, it enhances powder flow by reducing the inter-particle friction.

According to this embodiment of the present invention, the amount of lubricant in the tablet is 0.1% to 3.0% by weight of the total composition.

According to this embodiment of the present invention, the said tablet is used as a core tablet, which is then coated with a film coating to obtain a film-coated tablet form.

According to this embodiment of the present invention, the said tablet is used as mini-tablets, which is then filled into a capsule. Mini tablets of 0.25 mg dose are placed in different capsules, providing easy access to each dose brexpiprazole product.

According to this embodiment of the present invention, the tablet comprises;
▪ Brexpiprazole or a salt thereof having particle size d90 less than about 35 microns,
▪ Corn starch
▪ Crospovidone
▪ Sodium stearyl fumarate

According to this embodiment of the present invention, the tablet comprises;
▪ Brexpiprazole or a salt thereof having particle size d90 less than about 35 microns,
▪ Lactose monohydrate
▪ Microcrystalline cellulose
▪ Corn starch
▪ Crospovidone

According to this embodiment of the present invention, the tablet is prepared wet granulation which is simple and cost-effective method. Solvent is used in the process.

Suitable solvents are selected from the group comprising water, ethanol, dichloromethane, 0.1N HCl, isopropyl alcohol, benzyl alcohol, propylene glycol, polyethylene glycol, glycerine, glycerine triacetate, diethylene glycol monoethyl ether, glycerol, propylene carbonate or mixtures thereof.

According to this embodiment of the present invention, the solvent during wet granulation is water.

### Example 1: Tablet

| **Ingredients** | **Amount (% by weight of the total formulation)** | **Ingredients in CORE TABLET % by weight** | **Ingredients in MINI TABLET % by weight** |
|---|---|---|---|
| Brexpiprazole | 0.1 -10.0 | 3.33 | 1.00 |
| Lactose monohydrate | 40.0 - 60.0 | 56.11 | 56.80 |
| Microcrystalline cellulose | 5.0 - 25.0 | 11.11 | 12.00 |
| Corn starch | 10.0 - 32.0 | 22.22 | 23.00 |
| Polyvinylpyrrolidone | 1.0 -10.0 | 3.56 | 3.60 |
| Crospovidone | 1.0 -10.0 | 3.00 | 3.00 |
| Sodium stearyl fumarate | 0.1 -3.0 | 0.67 | 0.60 |
| Water | q.s. | q.s. | q.s. |
| **TOTAL** | **100** | **100** | **100** |

A process for example 1;
a) Mixing lactose monohydrate, corn starch and microcrystalline cellulose,
b) Mixing brexpiprazole and crospovidone,
c) Mixing the mixture at step (a) and the mixture at step (b) with geometric dilution
d) Sieving the mixture into 0.9 mm and then mixing,
e) Preparing a granulation solution with polyvinylpyrrolidone and water (preferably %10 PVP),
f) Granulating the mixture at step (d) with the granulation solution at step (e),
g) Sieving the mixture into 2 mm and then mixing,
h) Drying the mixture are fluid bed until humidity reaches under 2%,
i) Sieving the dry mixture into 1 mm and then mixing,
j) adding sodium stearyl fumarate and then mixing,
k) Compressing the total mixture into tablet.

Preferably, the tablet is present in the form of mini-tablets, and the mini tablets is filled into capsule.

Preferably, the tablet is coated with film coating to obtain film coated tablet.

### Example 2: Tablet

| **Ingredients** | **Amount (% by weight of the total formulation)** | **Ingredients in CORE TABLET % by weight** | **Ingredients in MINI TABLET % by weight** |
|---|---|---|---|
| Brexpiprazole | 0.1 -10.0 | 3.33 | 1.0 |
| Lactose monohydrate | 40.0 - 60.0 | 55.67 | 56.4 |
| Microcrystalline cellulose | 5.0 - 25.0 | 11.11 | 12.0 |
| Corn starch | 10.0 - 32.0 | 22.22 | 23.0 |
| Hydroxypropyl cellulose | 1.0-10.0 | 4.00 | 4.0 |
| Crospovidone | 1.0-10.0 | 3.00 | 3.0 |
| Sodium stearyl fumarate | 0.1 -3.0 | 0.67 | 0.6 |
| Water | q.s. | q.s. | q.s. |
| **TOTAL** | **100** | **100** | **100** |

A process for example 2;
a) Mixing lactose monohydrate, corn starch and microcrystalline cellulose,
b) Mixing hydroxypropyl cellulose, brexpiprazole and crospovidone,
c) Mixing the mixture at step (a) and the mixture at step (b) with geometric dilution,
d) Sieving the mixture into 0.9 mm and then mixing,
e) Adding water (about 30-35 gram) and granulating,
f) Sieving the mixture into 2 mm and then mixing,
g) Drying the mixture at fluid bed until humidity reaches under 2%,
h) Sieving the dry mixture into 1 mm and then mixing,
i) Adding sodium stearyl fumarate and then mixing,
j) Compressing the total mixture into a tablet.

Preferably, the tablet is present in the form of mini-tablets, and the mini tablets is filled into capsule.

Preferably, the tablet is coated with film coating to obtain film coated tablet.

## Claims

1. A tablet comprising brexpiprazole or a salt thereof and at least two fillers wherein the one of fillers is corn starch and brexpiprazole has a d (0.9) particle size between 1 µm and 35 µm.

2. The tablet according to claim 1, wherein brexpiprazole or a salt thereof has a d (0.9) particle size between 3 µm and 28 µm, more preferably between 5 µm and 18 µm.

3. The tablet according to claim 1, wherein used another fillers are selected from group comprising lactose monohydrate, microcrystalline cellulose, mannitol, pregelatinized starch, ammonium alginate, calcium carbonate, anhydrous lactose, calcium phosphate, calcium phosphate dehydrate, neutral pellets, calcium sulfate, cellulose, ethylcellulose, fructose, glyceryl palmitostearate, magnesium carbonate, magnesium oxide, maltodextrin, maltose, medium chain triglycerides, polydextrose, polymethacrylates, sodium alginate, sodium chloride, sorbitol, starch, sucrose, sugar sphericals, talc, polysorbate 80, xylitol or mixtures thereof.

4. The tablet according to claim 1 or 3, wherein another filler is lactose monohydrate, microcrystalline cellulose or mixtures thereof.

5. The tablet according to claim 1, wherein the tablet further comprises at least one pharmaceutically acceptable excipient which is selected from the group comprising binders, disintegrants, lubricants or mixtures thereof.

6. The tablet according to claim 5, wherein binders are selected from the group comprising polyvinylpyrrolidone, carboxymethylcellulose sodium, sugars, agar, alginates, carbomers, cellulose acetate phthalate, chitosan, starch, starch mucilage, acacia mucilage, dextrates, dextrin, dextrose, ethylcellulose, glyceryl behenate, hydrogenated vegetable oil type I, hydroxyethyl cellulose, hydroxyethylmethyl cellulose, hydroxypropyl cellulose, hydroxypropyl starch, hypromellose, magnesium aluminum silicate, maltodextrin, methylcellulose, poloxamer, polycarbophil, polydextrose, polyethylene oxide, polymethacrylates, aluminia hydroxide, stearic acid, sucrose, , polyoxyethilene-alkyl ethers, pullulan or mixtures thereof.

7. The tablet according to claim 5 or 6, wherein the binder is polyvinylpyrrolidone or hydroxypropyl cellulose or mixtures thereof.

8. The tablet according to claim 5, wherein disintegrants are selected from the group comprising sodium starch glycolate, crospovidone, cross-linked carboxymethyl cellulose (croscarmellose sodium), low-substituted hydroxypropyl cellulose, carboxymethyl cellulose, docusate sodium, guar gum, sodium alginate, alginic acid, magnesium aluminium silica, poloxamer, sodium glycine carbonate or mixtures thereof.

9. The tablet according to claim 5 or 8, wherein the disintegrant is crospovidone.

10. The tablet according to claim 5, wherein lubricants are selected from the group comprising sodium stearyl fumarate, magnesium stearate, calcium stearate, zinc stearate, talc, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyceryl palmito sulphate, sodium lauryl sulphate or mixtures thereof.

11. The tablet according to claim 5 or 10, wherein the lubricant is sodium stearyl fumarate.

12. The tablet according to claim 1, wherein the said tablet is used as a core tablet, which is then coated with a film coating to obtain a film-coated tablet form.

13. The tablet according to claim 1, wherein the said tablet is used as mini-tablets, which is then filled into a capsule.

14. The tablet according to claim 1 or 5, wherein the tablet comprises;
▪ Brexpiprazole or a salt thereof having particle size d90 less than about 35 microns,
▪ Corn starch
▪ Crospovidone
▪ Sodium stearyl fumarate

15. The tablet according to claim 1 or 5, wherein the tablet comprises;
▪ Brexpiprazole or a salt thereof having particle size d90 less than about 35 microns,
▪ Lactose monohydrate
▪ Microcrystalline cellulose
▪ Corn starch
▪ Crospovidone
